# EUROPEAN PATENT SPECIFICATION

(11) **EP 1 465 627 B1**
(45) Date of publication and mention of the grant of the patent: **08.03.2006**
(21) Application number: 02805961.6
(22) Date of filing: 18.12.2002
(51) Int. Cl.: A61K 31/4402, A61K 31/42, A61P 3/06, A61P 25/28, A61P 9/00

(54) **DOSING REGIMEN FOR PPAR−GAMMA ACTIVATORS**
DOSIERSCHEMA FÜR PPAR-GAMMA-AKTIVATOREN
MODE D'ADMINISTRATION D'ACTIVATEURS DU PPAR-GAMMA

(30) Priority: 21.12.2001 US 345153 P
(43) Date of publication of application: 13.10.2004
(73) Proprietor: SMITHKLINE BEECHAM CORPORATION, Philadelphia, PA 19101 (US)
(72) Inventor: BROWN, Kathleen, Keating, GlaxoSmithKline, Research Triangle Park, NC 27709 (US); BAER, Philip, G., Rougmont, NC 27572 (US)
(74) Representative: Learoyd, Stephanie Anne
(86) International application number: PCT/US2002/040699
(87) International publication number: WO 2003/055485

(56) References cited:
- WO-A-00/27341
- WO-A-00/28990
- WO-A-00/78333
- US-B1- 6 288 095
- US-B1- 6 294 580

## Description

### Field of the Invention

This invention relates to a new dosing regimens for PPAR-gamma activators.

### Background

Peroxisome Proliferator Activated Receptors (PPARs) are orphan receptors belonging to the steroid/retinoid receptor superfamily of ligand-activated transcription factors. See, for example, Willson, T. M. and Wahli, W., Curr. Opin. Chem. Biol., (1997), Vol. 1, pp 235-241. Three mammalian PPARs have been identified which are termed PPAR-alpha, PPAR-gamma, and PPAR-delta. PPARs regulate expression of target genes by binding to DNA response elements as heterodimers with the retinoid X receptor. These DNA response elements have been identified in the regulatory regions of a number of genes encoding proteins involved in glucose and lipid metabolism as well as energy balance. The biological role of the PPARs in the regulation of lipid metabolism and storage has been recently reviewed. See, for example, Spiegelman, B. M., Diabetes, (1998), Vol. 47, pp 507-514, Schoonjans, K., Martin, G., Staels, B., and Auwerx, J., Curr. Opin. Lipidol. (1997), Vol. 8, pp 159-166, and Brun, R. P., Kim, J. B., Hu, E., and Spiegelman, B. M., Curr. Opin. Lipidol. (1997), Vol. 8, pp 212-218, Way J.M., Harrington W. W., Brown K. K., Gottschalk W. K., Sundseth S. S., Mansfield T. A., Ramachandran R. K., Willson T. M. and Kliewer S. A., Endocrinology (2001), Vol. 142, pp1269-1277, Desvergne, B. Wahli, W Endocrinology Reviews (1999) 20(5): 649-688).

PPAR-gamma activators are typically used to treat type 2 diabetes. PPAR-gamma activators enhance sensitivity to insulin-stimulated glucose uptake and utilization, and thus reduce plasma glucose levels in human diabetics and in rodent models of diabetes. This sustained plasma glucose lowering effect, reflected clinically as a decrease in HbAlc, is referred to as "glycemic control", and represents the desired anti-diabetic efficacy of this compound class. Additional benefits of PPAR-gamma activator treatment in diabetic patients are reduction in serum triglyceride and free fatty acid levels, decrease in LDL cholesterol and an increase in HDL cholesterol.

However, the benefits of treatment with PPAR-gamma activators can be accompanied by undesired side effects. A significant dose-limiting side effect of the currently marketed PPAR-gamma activators, observed in diabetic patients, is weight gain due to fluid retention. This fluid retention may lead to edema and, particularly in patients with pre-existing heart failure, the development of congestive symptoms,. The incidence of edema may be increased if the PPAR-gamma activator) is used in combination either with agents that stimulate insulin secretion, for example sulfonylureas, or exogenously administered insulin. See, for example, ACTOS, Physicians Desk Reference, (2001) 55^{th} edition, pp. 3171-3175 or AVANDIA Physicians Desk Reference, (2001) 55^{th} edition, pp. 3871-3875.

Drug dosing regimens are typically designed to be convenient for the patient, to promote compliance, to produce maximal benefit, and to minimize undesired side effects. For the vast majority of drugs, this is best achieved through constant drug exposure at the lowest level that produces efficacy, and thus the optimal regimen is in most cases daily drug administration. For drugs given daily, efficacy and side effects are generally separable by total drug dosage, with side effects occurring at drug dosages that are higher by some multiple than the dosage producing the desired efficacy. Currently, PPAR-gamma activators are administered once or twice daily. See, for example ACTOS, (Physicians Desk Reference, (2001) 55^{th} edition, p 3175 or AVANDIA, Physicians Desk Reference, (2001) 55^{th} edition, p 3875.

In addition to daily drug dosing, there are examples of drugs that use dosing regimens in which the interval between drug doses is more than a single day. While the interval may be two days (i.e. alternate-day dosing), or one week (i.e. once weekly dosing), or an extended period of no dosing following a period of daily dosing, the underlying principles are the same. In this discussion, any regimen intentionally comprising an inter-dose interval greater than one day is referred to as less than daily dosing.

Less than daily dosing is discussed extensively in therapeutic areas in which glucocorticoid therapy is a mainstay. See, for example, Axelrod L., Glucocorticoids, Textbook of Rheumatology, 4^{th} edition, vol 1, pp 779-ff, (1986); Physicians Desk Reference, 48^{th} edition, section on DELTASONE, pp 2408-2409, (1994); Holland and Taylor, Glucocorticoids in clinical practice, J Family Prac 32: 512-519, (1991); and Hodson EM, Knight JR, Willis NS, and Craig JC, Corticosteroid therapy for nephrotic syndrome in children (Cochrane Review), The Cochrane Library, Issue 4, (2001).

For hormone replacement therapy, for treatment of menopausal disorders, estrogen has been administered by a variety of dosing regimens, often given daily for several weeks, followed by one week of no dosage. This is intended to minimize stimulation of proliferation in estrogen-sensitive tissues, such as uterus, by partly mimicking the normal endocrine physiology. See, for example The Pharmacological Basis of Therapeutics, (1996) 9^{th} edition, pp. 1422-1423.

Alendronate, used for prevention and treatment of osteoporosis, was originally developed for daily dosing. Subsequently, for various reasons, including minimization of esophagitis seen as a side effect, the drug was approved for once-a-week dosing. In this case, knowledge of pharmacokinetics, specifically that the drug distributes only into bone, was used to predict that infrequent dosing would be fully efficacious, since bone is the target tissue for the drug. See, for example Schnitzer, TJ, Update on Alendronate: Once-Weekly dosing. Expert Opin Pharmacother 2: 1461-1472, (2001).

In addition to minimization of side effects, minimization of drug cost is sometimes part of the rationale for alternate-day dosing. For treatment of hypercholesterolemia, it was recently reported that lovastatin given as less than daily dosing was equally as efficacious as when given daily, and thus was a cost-effective method of treatment. See, for example Dennis, VC, Britton ML, Sirmans SM, Letassy NA, Freeman DA, The use of alternate-day lovastatin in hypercholesterolemic men, Ann Pharmacother 31: 708-711, (1997).

### Brief Description of the Invention

Briefly, in one aspect, the present invention relates to a method of treating a disease or condition, for example diabetes, with a PPAR-gamma activator comprising administration of the PPAR-gamma activator according to a dosing schedule that comprises a less than daily dosing of the activator, for example alternate day dosing. The dosing regimen of this invention, as compared to dosing regimens comprising daily or twice daily administration of PPAR-gamma activator, may result in no significant decrease in efficacy, for example glycemic control, but with a significant decrease in undesired effects, for example fluid retention which can lead to weight gain, hemodilution, and edema. The dosing regimen of this invention may be used in conjunction with other treatments. For example, the PPAR-gamma activator dosing regimen of this invention can be used in combination with either exogenous insulin or drugs that increase the secretion of endogenous insulin.

### Detailed Description of the Invention ,

PPAR-gamma activators includes PPAR-gamma agonists. As used herein, a "PPAR-gamma activator" includes any compound that activates human PPAR-gamma by any accepted assay, or any compound generally recognized as a PPAR-gamma activator or agonist. Such PPAR-gamma activators may be activators of more than one PPAR subtype. Preferred PPAR-gamma activators include the thiazolidinediones (TZDs) known to be useful for treating diabetes and also non-thiazolidinedione (non-TZDs) PPAR-gamma activators such as those disclosed in US Patent 6,294,580. Particularly preferred TZDs include those currently marketed, such as rosiglitzone and pioglitazone. Particularly preferred non-TZDs include compounds in development such as the GlaxoSmithKline compound G1262570 (farglitazar).

As used herein "less than daily dosing" will be used to indicate any intentional dosing regimen that comprises a frequency of dosing which is less than daily, for example every other day, or which comprises at least one gap of at more than 1 day where there is no administration of the PPAR-gamma activator. In the case of dosing regimens that comprise a gap or day without administration, as used herein such gaps or days without administration of the PPAR-gamma activator must be preceded and followed by administration of activator. This is meant to include any dosing regimen comprising gaps in dosing of more than one day. For example, the dosing regimens of this invention include regimens comprising 5 days with and 2 days without administration of the activator, or 12 with and 2 without, or 19 with and 2 without, or 26 with and 2 without, or 11 with and 3 without, or 18 with and 3 without, or 25 with and 3 without, or 10 with and 4 without, or 17 with and 4 without, or 24 on and 4 without, etc. This is true regardless of the average number of doses per day. For example, twice daily administration every other day would be less than daily dosing. The dosing regimen of this invention can include several days or weeks of daily or twice daily dosing followed by a period of less than daily dosing. As used herein a day with dosing or a day with administration of activator includes once, twice, or any number of doses on that particular day.

Preferred dosing regimens are those that comprise periods of every other day or every third day or twice weekly dosing, or that comprise one or two or three consecutive days without administration. When the PPAR-gamma activator dosing regimen of this invention is used in combination with administration of either exogenous insulin or drugs that increase the secretion of endogenous insulin, the insulin or additional drug can be administered with a dosing regimen that is the same as or different from the PPAR-gamma activator regimen. As used herein "dosing" and "administration" are intended to be identical.

### Experimental

Four animal studies were carried out regarding the effects of PPAR-gamma activator treatment. The PPAR-gamma activator used in these experiments was GI262570 (farglitazar), which may be prepared as described in US Patent 6,294,580. These four studies are described in detail below. As used herein "bid" means twice daily.

### Study 1: Rat Model of PPAR-gamma Activator Induced Fluid Retention/Edema

Normal Han Wistar female adult rats were housed in metabolic cages and orally dosed (8 mg/kg, bid) with PPAR-gamma activator or vehicle (PEG400, 1 ml/kg) for 21 days. Electrolyte and water balance, plasma volume, hematocrit, and interstitial fluid volume were measured. Compared with vehicle treated control rats, rats treated with PPAR-gamma activator showed a sustained increase in electrolyte and water retention, ultimately resulting in a 6% increase in plasma volume and a 6% decrease in hematocrit. In addition, there was a 50% increase in interstitial fluid volume, correlating to an edematous state.

These results indicate that daily treatment with a PPAR-gamma activator caused sustained fluid retention and development of edema. As noted in the background section above, this PPAR-gamma activator effect has also been observed in human clinical studies.

### Study 2: Rat Model of PPAR-gamma Activator Induced Cardiovascular Changes Leading to Fluid Retention and Edema

Normal Han Wistar female adult rats were chronically instrumented for measurement of cardiac output, blood pressure and total peripheral resistance and orally dosed (8 mg/kg, bid) with PPAR-gamma activator or vehicle (PEG400, 1 ml/kg) for 10 days. Data were collected for 7 days prior to the start of dosing, for 10 days of dosing, and for 7 days after discontinuation of dosing. In rats treated with the PPAR-gamma activator, total peripheral resistance decreased, and cardiac output increased, beginning within six hours after the initial dose, and reached steady state by treatment day four. When dosing was discontinued, total peripheral resistance and cardiac output began to return toward control values within 24 hours, with full recovery after four days. The decrease in total peripheral resistance is believed to be the initial pharmacodynamic effect of PPAR-gamma activators leading to fluid retention and edema.

### Study 3: Model of PPAR-gamma Activator Induced Efficacy/Glycemic Control

Two glucose-matched groups (n=6/group) of Zucker Diabetic Fatty (ZDF) rats, 7-8 weeks of age, were treated with vehicle (n-methylglucamine, 0.05M, 1 ml/kg, p.o.) or PPAR-gamma activator (5 mg/kg, bid). After four weeks of treatment, all rats treated with PPAR-gamma activator were volume expanded and two were overtly edematous, and PPAR-gamma activator dosing was discontinued. At the time dosing was discontinued, the average non-fasted plasma glucose was 154 mg/dl in PPAR-gamma agonist treated rats compared to 464 mg/dl in the vehicle-treated controls. This level of glycemic control was maintained despite discontinuation of PPAR-gamma activator treatment for 10 days, at which time the average plasma glucose in the rats previously treated with PPAR-gamma activator was 175 mg/dl, compared to 462 mg/dl in vehicle-treated controls. In contrast to the sustained glycemic control, the fluid volume expansion was not sustained, as evidenced by the finding that the hematocrit returned from and end-of-treatment value of 47% to a control value or 52% within 3 days.

Taking the hematocrit response as indicative of the expanded plasma volume resulting from increased fluid and electrolyte retention, these data suggested that the beneficial pharmacodynamic effect of PPAR-gamma activators (glycemic control) was of longer duration than the pharmacodynamic effects leading to salt and water retention. In other words, the results above show that the time course for the cardiovascular changes that lead to the reversal of the fluid retention and edema was shorter than the duration of glycemic control after discontinuation of dosing. This time-differential finding suggests that it may be possible to achieve glucose control without fluid retention during PPAR-gamma activator therapy by decreasing the frequency of dosing from daily to some form of less than daily dosing. The results also show that the mechanism of PPAR-gamma induced fluid retention, namely the fall in total peripheral resistance, begins within six hours of the first dose, a vascular effect that will stimulate salt and water retention. Knowledge of the on and off rate of the primary vascular and secondary renal effects suggests an appropriate dosing regimen. This was tested in Study 4 below.

### Study 4: Less Than Daily Dosing with PPAR-gamma Activator

Three groups of 8 plasma glucose-matched ZDF rats with non-fasted glucose values greater than 250 mg/dl were dosed with PPAR-gamma activator. During the study plasma glucose and serum albumin were measured weekly, HbAlc was measured every other week, and heart weights and body weights were measured terminally. Reduction of plasma glucose and HbA1c are markers for anti-diabetic efficacy (glycemic control). Decreased plasma albumin concentration is a marker for plasma volume expansion or hemodilution. The heart weight-to-body weight ratio is a marker for increased cardiac output secondary to volume expansion.

All three groups were initially dosed with PPAR-gamma activator for one week at 5 mg/kg twice daily, in order to achieve glycemic control as shown by plasma glucose less than 250 mg/dl, and volume expansion as shown by reduced serum albumin.

After the initial week of dosing, the mean non-fasted plasma glucose for all rats was 138 mg/dl (compared to pre-treatment values of 321 mg/dl) and the serum albumin concentration was 3.5 gm/dl (compared to pre-treatment value of 3.8 gm/dl). Following the first week one group (Group BID) was continued on the daily dosing regimen while the other two were moved to less than daily dosing. Group BID continued to be dosed at 5 mg/kg twice daily, Group MWF was dosed at 10 mg/kg per day three times weekly (Monday/Wednesday/Friday), and Group MF was dosed at 10 mg/kg per day twice weekly (Monday/Friday). These three dosing regimens were continued for five weeks.

Because this study was designed to determine whether fluid retention could be eliminated while glycemic control was maintained, only animals demonstrating continued glycemic control (non-fasted plasma glucose less than 250 mg/dl) were evaluated. All 8 rats in the BID group met this criterion, as did 6 in the MWF group and 5 in the MF group. The data is summarized in Tables 1, 2, and 3, and only includes the 19 rats that exhibited glycemic control.

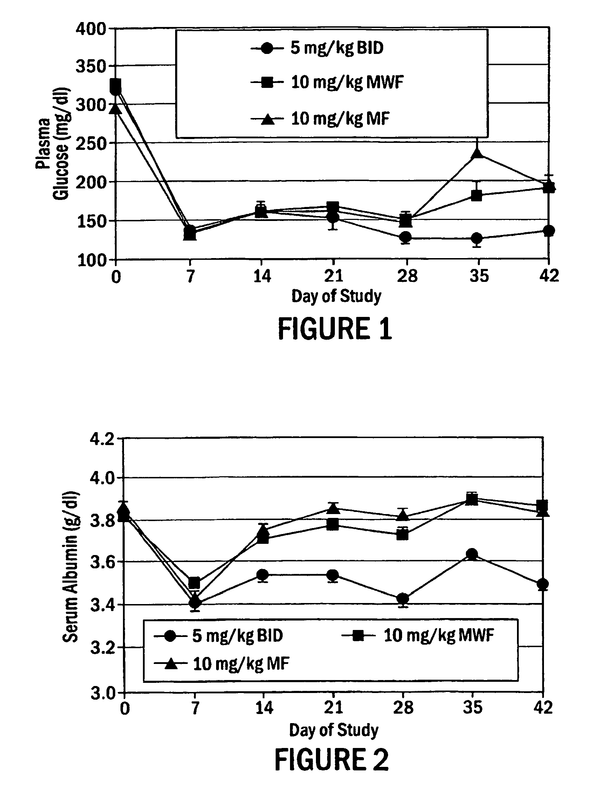

The data summarized in Figure 1 show that during the last two weeks of study, plasma glucose (in the 11 responding rats) was slightly higher in the MWF and MF dose groups, but still well below pre-dosing levels, thus demonstrating that all three dosing regimens resulted in glycemic control in these animals.

As further evidence of this, HbA_{1c} values, which indicate average plasma glucose integrated over time, were below 6% for all 19 animals. The data summarized in Figure 2 show that serum albumin concentration was similarly decreased in all rats after the first week of dosing, indicating that plasma volume was increased. By day 14 of reduced frequency dosing, the MWF and MF group values returned to and remained at pre-treatment baseline values, while the BID group value remained below the pre-treatment baseline value throughout dosing.

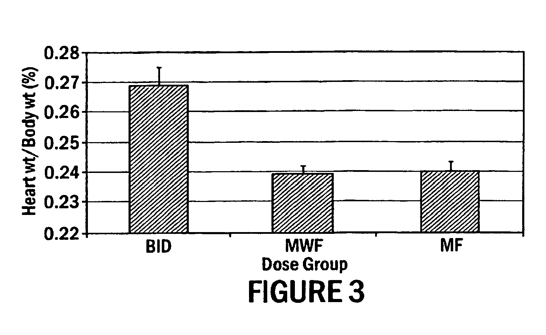

The data summarized in Figure 3 show that the terminal heart weight to body weight ratio for the BID group was increased by 13% compared to the MWF and MF dose groups. Heart weight/body weight ratio values from the literature for normal rats of the same body weight average 0.23 with a range of 0.19-0.25 as the 95% confidence limits. Thus, both the MWF and MF dose group heart weight/body weight ratios were within normal ranges, while that for the BID dose group was increased beyond the normal range. This indicates that the alternate day dosing regimens prevented the fluid retention that occurs with daily dosing, and thus prevented the increase in cardiac output that leads to increased heart weight.

The results of the studies described above demonstrate that, with alternate day dosing regimens, it is possible to achieve the desired glycemic control with PPAR-gamma activators, without producing undesired fluid retention and edema.

## Claims

1. Use of a PPAR-gamma activator in the manufacture of a medicament for treating a disease or condition for administration of the PPAR-gamma activator according to a dosing schedule which comprises a period of less than daily dosing.

2. Use according to Claim 1 wherein the dosing schedule comprises at least one day in which there is no administration of the PPAR-gamma activator.

3. Use according to Claim 1 wherein the dosing schedule comprises 2 consecutive days in which there is no administration of the PPAR-gamma activator.

4. Use according to Claim 1 wherein the dosing schedule comprises a period selected from the group consisting of administration of the activator on alternate days, administration of the activator on only two days per week, and administration of the activator every third day.

5. Use according to Claims 1-4 wherein the PPAR-gamma activator is rosiglitazone or fargl itazar.

6. Use according to Claims 1-5 wherein the disease or condition is selected from the group consisting of hyperglycaemia, dyslipidemia, Type II diabetes, Type I diabetes, hypertriglyceridemia, syndrome X, insulin resistance, heart failure, diabetic dyslipidemia, hyperlipidemia, hypercholesteremia, hypertension, obesity, anorexia bulimia, anorexia nervosa, and cardiovascular disease, including ischemia-reperfusion injury and atherosclerosis, cancer, Alzheimer's disease or other cognitive disorders.

7. Use according to Claim 6 wherein the disease or condition is diabetes.

8. Use according to claim 7 further comprising administration of an agent that stimulates insulin secretion or insulin.

9. Use according to Claim 8 wherein said agent is selected from the group consisting of sulfonyureas, metformin, glucophage.

## Patentansprüche

1. Verwendung eines PPAR-gamma-Aktivators in der Herstellung eines Medikaments zur Behandlung einer Krankheit oder eines Zustands zur Verabreichung des PPAR-gamma-Aktivators gemäß einem Dosierungsschema, das eine Periode einer weniger als täglichen Dosierung umfaßt.

2. Verwendung gemäß Anspruch 1, worin das Dosierungsschema wenigstens einen Tag umfaßt, an dem es keine Verabreichung des PPAR-gamma-Aktivators gibt.

3. Verwendung gemäß Anspruch 1, worin das Dosierungsschema zwei aufeinanderfolgende Tage umfaßt, an denen es keine Verabreichung des PPAR-gamma-Aktivators gibt.

4. Verwendung gemäß Anspruch 1, worin das Dosierungsschema eine Periode umfaßt, die aus der Gruppe ausgewählt ist, die aus der Verabreichung des Aktivators an abwechselnden Tagen, der Verabreichung des Aktivators an nur zwei Tagen pro Woche und der Verabreichung des Aktivators an jedem dritten Tag besteht.

5. Verwendung gemäß Ansprüchen 1 bis 4, worin der PPAR-gamma-Aktivator Rosiglitazon oder Farglitazar ist.

6. Verwendung gemäß Ansprüchen 1 bis 5, worin die Krankheit oder der Zustand aus der Gruppe ausgewählt ist, die aus Hyperglykämie, Dyslipidämie, Typ II-Diabetes, Typ I-Diabetes, Hypertriglyceridämie, Syndrom X, Insulinresistenz, Herzversagen, diabetischer Dyslipidämie, Hyperlipidämie, Hypercholesterinämie, Hypertonie, Fettsucht, Anorexia bulimia, Anorexia nervosa und kardiovaskulärer Krankheit, einschließlich Ischämie-Reperfusionsverletzung und Atherosklerose, Krebs, Alzheimer-Krankheit oder anderen kognitiven Störungen besteht.

7. Verwendung gemäß Anspruch 6, worin die Krankheit oder der Zustand Diabetes ist.

8. Verwendung gemäß Anspruch 7, die ferner die Verabreichung eines Mittels umfaßt, das die Insulinsekretion oder Insulin stimuliert.

9. Verwendung gemäß Anspruch 8, worin das Mittel aus der Gruppe ausgewählt ist, die aus Sulfonylharnstoffen, Metformin und Glucophage besteht.

## Revendications

1. Utilisation d'un activateur du PPAR-gamma dans la fabrication d'un médicament destiné à traiter une maladie ou une affection pour l'administration de l'activateur du PPAR-gamma selon un mode d'administration qui comprend une fréquence inférieure à une fois par jour.

2. Utilisation selon la revendication 1, dans laquelle le mode d'administration comprend au moins un jour sans administration de l'activateur du PPAR-gamma.

3. Utilisation selon la revendication 1, dans laquelle le mode d'administration comprend 2 jours consécutifs sans administration de l'activateur du PPAR-gamma.

4. Utilisation selon la revendication 1, dans laquelle le mode d'administration comprend une fréquence choisie dans le groupe constitué par l'administration de l'activateur tous les deux jours, l'administration de l'activateur uniquement deux jours par semaine, et l'administration de l'activateur tous les trois jours.

5. Utilisation selon les revendications 1 à 4, dans laquelle l'activateur du PPAR-gamma est la rosiglitazone ou le farglitazar.

6. Utilisation selon les revendications 1 à 5, dans laquelle la maladie ou l'affection est choisie dans le groupe constitué par l'hyperglycémie, la dyslipidémie, le diabète de type II, le diabète de type I, l'hypertriglycéridémie, le syndrome X, l'insulinorésistance, l'insuffisance cardiaque, la dyslipidémie diabétique, l'hyperlipidémie, l'hypercholestérolémie, l'hypertension, l'obésité, l'anorexie-boulimie, l'anorexie mentale, et les maladies cardiovasculaires, notamment la lésion d'ischémie-reperfusion et l'athérosclérose, le cancer, la maladie d'Alzheimer ou autres troubles cognitifs.

7. Utilisation selon la revendication 6, dans laquelle la maladie ou l'affection est le diabète.

8. Utilisation selon la revendication 7 comprenant en outre l'administration d'un agent qui stimule la sécrétion d'insuline ou l'insuline.

9. Utilisation selon la revendication 8, dans laquelle ledit agent est choisi dans le groupe constitué par les sulfonylurées, la metformine, le glucophage.
